(19) Europäisches Patentamt
European Patent Office
Office européen des brevets

(11) EP 2 104 428 B1

(12) **EUROPÄISCHE PATENTSCHRIFT**

(45) Veröffentlichungstag und Bekanntmachung des Hinweises auf die Patenterteilung:
**14.10.2015 Patentblatt 2015/42**

(21) Anmeldenummer: **07856722.9**

(22) Anmeldetag: **14.12.2007**

(51) Int Cl.:
***A01N 53/00*** *(2006.01)* ***A01N 47/02*** *(2006.01)*
***A01N 43/56*** *(2006.01)* ***A01N 25/02*** *(2006.01)*
***A01P 7/02*** *(2006.01)* ***A01P 7/04*** *(2006.01)*
***A61K 31/415*** *(2006.01)* ***A61K 47/08*** *(2006.01)*
***A61K 47/22*** *(2006.01)* ***A01N 43/28*** *(2006.01)*

(86) Internationale Anmeldenummer:
**PCT/EP2007/010981**

(87) Internationale Veröffentlichungsnummer:
**WO 2008/080542 (10.07.2008 Gazette 2008/28)**

(54) **KOMBINATIONSPRODUKT ZUR BEKÄMPFUNG VON PARASITEN AN TIEREN**
COMBINATION PRODUCT FOR CONTROLLING PARASITES ON ANIMALS
PRODUIT DE COMBINAISON DESTINÉ À LA LUTTE CONTRE LES PARASITES CHEZ L'ANIMAL

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**HR**

(30) Priorität: **27.12.2006 DE 102006061538**

(43) Veröffentlichungstag der Anmeldung:
**30.09.2009 Patentblatt 2009/40**

(73) Patentinhaber: **Bayer Intellectual Property GmbH**
**40789 Monheim (DE)**

(72) Erfinder:
- **SIRINYAN, Kirkor**
  **51467 Bergisch Gladbach (DE)**
- **TURBERG, Andreas**
  **42781 Haan (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**WO-A-96/16543** **WO-A-2004/098290**
**WO-A-2005/048713** **US-A- 5 516 787**
**US-A1- 2003 187 029** **US-B1- 6 395 765**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 104 428 B1

## Beschreibung

Die Erfindung betrifft neue Mittel zur Bekämpfung von Parasiten an Tieren, enthaltend ein N-Arylpyrazol sowie ein Pyrethroid in einer Formulierung enthaltend aliphatische, cyclische Carbonate und aliphatische cyclische oder acyclische Polyether.

N-Arylpyrazole sowie ihre gute insektizide und akarizide Wirksamkeit sind bekannt aus US 2006014802 A1, WO2005090313 A1, FR2834288 A1, WO09828277, US06069157, WO200031043, DE19824487, WO09804530, WO09962903, EP00933363, EP00911329,WO09856767, US05814652, WO09845274, WO9840359, WO09828279, WO09828278, DE19650197, WO09824767, EP00846686, EP00839809, WO09728126, EP00780378, GB02308365, US05629335, WO09639389, US05556873, EP00659745, US05321040, EP00511845, EP-A-234119, EP-A-295117 und WO 98/24769. Trotz dieser Fülle von Anmeldungen mit zahlreichen N-Arylpyrazol Strukturen gibt es einen überlegenen Strukturtyp der in der Mehrzahl der Indikationen im Vergleich die beste Wirksamkeit zeigt. 1-[2,6-Dichloro-4-(trifluoromethyl)phenyl]-3-cyano-4-[(trifluoromethyl)sulfinyl]-5-aminopyrazol (INN: Fipronil) ist allgemein anerkannt als wirksamste Verbindung in dieser Klasse bei der Bekämpfung der meisten Parasiten.

Seit über 10 Jahren sind N-Arylpyrazole als Ektoparasitizide im Markt (Hunter, J. S., III, D. M. Keister and P. Jeannin. 1994. Fipronil: A new compound for animal health. Proc. Amer. Assoc. Vet. Parasitol. 39th Ann. Mtg. San Francisco, CA. Pg. 48.). Sie zeichnen sich durch gute und breite Wirksamkeit und akzeptable Verträglichkeit aus. Es ist bekannt, dass die bestehenden Formulierungen mit einem hohen Anteil an DEE (Transcutol) eine starke transdermale (FR 1996-11446 A; Sicherheitsdatenblatt: ISO/DIS 11014/29 CFR 1910.1200/ANSI Z400.1 Printing date 10/23/2001: FRONTLINE® TOP SPOT™: Fipronil 9.7% w/w) Komponente enthalten. Damit wird über die Formulierung das Eindringen in die Talgdrüsen und die Epithelschichten erleichtert (Skin distribution of fipronil by microautoradiography following topical administration to the beagle dog. Cochet, Pascal; Birckel, P.; Bromet-Petit, M.; Bromet, N.; Weil, A.; European Journal of Drug Metabolism and Pharmacokinetics (1997), 22(3), 211-216.). Eine hohe Konzentration in den Talgdrüsen kann über die Sebumexkretion aus den Talgdrüsen zu einer lang anhaltenden Wirkstoffverfügbarkeit beitragen, wenn der Wirkstoff mitgeschleppt wird. Dennoch ist bei den üblichen Formulierungen eine Penetration von N-Arylpyrazolen auch in den Blutkreislauf wahrscheinlich, da jedes Haarfollikel von einem Blutgefäß versorgt wird und so nur eine sehr dünne Barriere die Follikel vom Blutkreislauf trennt (Transfollicular drug delivery - Is it a reality? Meidan, Victor M.; Bonner, Michael C.; Michniak, Bozena B.; International Journal of Pharmaceutics (2005), 306(1-2), 1-14). Somit ist auch die Verfügbarkeit des Wirkstoffes auf dem Tier zeitlich und in der Konzentration begrenzt, da der Wirkstoff in den Blutkreislauf übertritt und somit seine verfügbare Konzentration im Sebum sinkt.

Weiter ist bekannt, dass die Wirksamkeit der N-Arylpyrazole bei Vertretern der Gattung Ixodes eine schwächere Wirkung zeigt als gegen andere Zeckengattungen (Endris RG, Matthewson, Cooke D & Amodie D (2000). Repellency and efficacy of 65% permethrin and 9.7% fipronil against Ixodes ricinus. Vet Therapeutics Vol. 1 (No, 3): 159-168); Endris RG, Cooke D, Amodie D, Sweenwy DL & Katz TL (2002). Repellency and efficacy of 65% permethrin and selamectin spot-on formulations against Ixodes ricinus on dogs. Vet Terapeutics. Vol. 3 (No. 1): 64-71.)

Pyrethroide haben zwar ebenfalls eine relativ breite insektizide Wirkung und einige Vertreter zeigen auch gute akarizide Effekte, allerdings kommt es bei diesen häufig zu Unverträglichkeiten und nur besonders untoxische Vertreter mit beschränkter Wirksamkeit können bei Katzen angewendet werden. Mit WO 04/098290 wurde kürzlich eine Lösung für dieses Problem beschrieben, bei dem mit Hilfe eines Synergisten, eines akariziden Pyrethroids sowie eines Neonicotinoids eine katzenverträgliche Dosierung erreicht werden konnte. Die unterschiedlichen physikochemischen Eigenschaften der Einsatzstoffe erfordern spezielle Formulierungen.

Weiterhin ist allgemein bekannt, dass Pyrethroide im Vergleich mit N-Arylpyrazolen bei der Aktivität gegenüber Zecken der Gattung Dermacentor schwächer abschneiden. Es wurde kürzlich weiter gefunden, dass es zwischen Pyrethroiden und N-Arylpyrazolen bei pyrethroidresistenten Insekten keine Kreuzresistenz gibt. Umgekehrt führt die Selektion solcher Mücken-Stämme mit N-Arylpyrazolen sogar zu einer teilweisen Revertierung der Pyrethroidresistenz.

[Laboratory evaluation of fipronil, a phenylpyrazole insecticide, against adult Anopheles (Diptera: Culicidae) and investigation of its possible cross-resistance with dieldrin in Anopheles stephensi. Kolaczinski, Jan; Curtis, Chris. London School of Hygiene and Tropical Medicine, London, UK. Pest Management Science (2001), 57(1), 41-45].

WO 2001/065941 A1 und EP 1013170 A1 schlagen die Kombination eines an N-Arylpyrazols und eines Pyrethroids in Anwendungen gegen Pflanzenschädlinge vor. JP 11049618 A2 verwendet ähnliche Mischungen um Fraßschäden an Holzkonstruktionen zu verhindern. WO 95/22902 A1 bekämpft mit solchen Mischungen Termiten direkt. FR 2713891 A1 und WO 95/22902 A1 beanspruchen sogar einen synergistischen Effekt derartiger Mischungen ohne dies aber klar nachzuweisen.

Aus [Antagonism of fipronil toxicity by piperonyl butoxide and S,S,S-tributyl phosphorotrithioate in the German cockroach (Dictyoptera: Blattellidae). Valles, Steven M.; Koehler, Philip G.; Brenner, Richard J. Center for Medical, Agricultural and Veterinary Entomology, USDA-ARS, Gainesville, FL, USA. Journal of Economic Entomology (1997), 90(5), 1254-1258] geht jedoch hervor, dass Inhibitoren des oxidativen Metabolismus (P450-oxidase Inhibitoren) in Schaben einen antagonistischen Effekt auf die Wirkung von N-Arylpyrazolen haben. Da die meisten Pyrethroide über

den p450-Oxidase-Weg detoxifiziert werden, sind diese ebenso wie MGK264 oder Piperonylbutoxid eher als Antagonisten denn als Synergisten der N-Arylpyrazole zu werten.

GB2396557 A1 lehrt, dass die Behandlung von Ektoparasiten mit Mischungen von N-Arylpyrazolen und Pyrethroiden (gegebenenfalls auch unter Zusatz von Synergisten wie MGK264 oder Piperonylbutoxid) möglich ist, wenn konzentrierte Pulverformulierungen eingesetzt werden. WO 95/22902 A1 beschreibt eine Bodenbehandlung mit verbesserter Wirkung durch kombinierten Einsatz von Phenylpyrazolen und Pyrethroiden zur Termitenkontrolle. Auch hier ist die verwendete Mischung für die Anwendung am Warmblüter ungeeignet.

WO96/16544 A2 offenbart emulgierbare Mittel, die bestimmte Phenylpyrazole und Pyrethroide enthalten.

WO2004/098290 A1 beschreibt neue, hautverträgliche, dermal anwendbare Flüssigformulierungen, die ein Pyrethrin oder ein Pyrethroid und MGK 264 in einem bestimmten Verhältnis sowie ein zusätzliches Insektizid enthalten.

US 6395765 beschreibt Formulierungen für Phenylpyrazole in Form von Lösungen und nennt unter anderem aliphatische Polyether als geeignete Lösungsmittel.

WO2005048713 offenbart neue insektizide Wirkstoffkombinationen von Anthranilsäureamiden mit Pyrethroid-Insektiziden sowie Mittel enthaltend diese Kombinationen.

Da derartige Formulierungen in der Praxis nur schwer applizierbar sind und durch die Partikel (GB 2396557) zusätzliche toxikologische Risiken bergen, muss es das Ziel sein, eine selbstspreitende Flüssigformulierung mit hoher Anwendersicherheit herzustellen, die die positiven Wirkeigenschaften der Pyrethroide mit denen der N-Arylpyrazole verknüpft und selbst in Anwesenheit weiterer Synergisten aus der Klasse der p450 Oxidaseinhibitoren zu keiner Abschwächung der N-Arylpyrazolwirksamkeit führt.

Dazu wurde durch intensive Analysen und Versuchsreihen aus einer Vielzahl von Additiven, Lösemitteln und Spreitmitteln nun Formulierungen identifiziert, welche die guten arthropodiziden Wirksamkeitseigenschaften der N-Arylpyrazole in Kombination mit Pyrethroiden im Allgemeinen verbessern können. Überraschenderweise wurden dabei die zu erwartenden antagonistischen Effekte nicht beobachtet.

Die Erfindung betrifft neue Mittel zur Bekämpfung von Parasiten an Tieren, enthaltend ein N-Arylpyrazol und ein Pyrethroid in einer Formulierung enthaltend:

- ein aliphatisches, cyclisches Carbonat
- einen aliphatischen cyclischen oder acyclischen Polyether sowie
- einen oder mehrere Ester eines zwei- oder dreiwertigen Alkohols mit bis zu drei Kohlenstoffatomen mit organischen Fettsäuren mit 6 bis 18 Kohlenstoffatomen

Die erfindungsgemäßen arthropodiziden Mittel sind neu und haben gegenüber den bisher beschriebenen Formulierungen eine wesentlich verbesserte und länger anhaltende Wirksamkeit bei gleichzeitig erhöhter Anwender- und Zieltiersicherheit.

In den Mitteln werden als Kombinationspartner der N-Arylpyrazole bevorzugt arthropodizide Pyrethroide, insbesondere der Typen Cyanopyrethroid (z.B. Flumethrin), Typ-1 Pyrethroid (z.B. Permethrin) oder Nicht-Ester Pyrethroide (Etofenprox) eingesetzt.

$\alpha$-Cyanopyrethroide (z.B. alpha-Cypermethrin, Cyfluthrin, beta-Cyfluthrin, Cyhalothrin, Cypermethrin, Deltamethrin, Fenvalerate, Flucythrinate, Flumethrin, tau-Fluvalinate) werden dabei bevorzugt im Konzentrationsbereich von 0.01 bis 5 Gew.-% eingesetzt und gegebenenfalls mit einem Synergisten versetzt (wie z. B. in WO 04/098290 beschrieben). Besonders bevorzugt werden Cypermethrin, Cyfluthrin, Deltamethrin und Flumethrin verwendet in einem Konzentrationsbereich von 0.025 bis 0.25 Gew.-%. Ganz besonders bevorzugt ist der Einsatz von Flumethrin in einem Konzentrationsbereich von 0.05 bis 1.25 Gew.-%.

Typ 1 Pyrethroide (z. B. Allethrin, Bioallethrin, Permethrin, Phenothrin, Resmethrin, Tetramethrin, Transfluthrin) werden bevorzugt in einem Konzentrationsbereich von 20 bis 70 Gew.-% eingesetzt. Besonders bevorzugt sind hier Permethrin, Cyphenothrin im Konzentrationsbereich von 30 bis 60 Gew.-%. Ganz besonders bevorzugt wird Permethrin in Konzentrationen von 40 bis 50Gew.-% eingesetzt.

Nicht-Esterpyrethroide (z. B. Etofenprox, Halfenprox, Silafluofen) werden üblicherweise in einem Konzentrationsbereich von 10 bis 60 Gew.-% eingesetzt. Bevorzugt sind Etofenprox oder Halfenprox, besonders bevorzugt ist Etofenprox im Konzentrationsbereich von 25-55%.

N-Arylpyrazole sind dem Fachmann als arthropodizide Wirkstoffe an sich bekannt, z. B. aus den oben genannten Dokumenten, auf die hiermit Bezug genommen wird.

Bevorzugte Phenylpyrazole sind diejenigen der Formel (I):

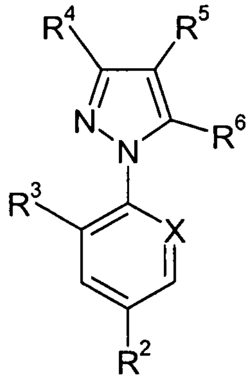

(I)

worin

X =N- oder C-$R^1$ bedeutet

$R^1$ und $R^3$ unabhängig voneinander für Halogen stehen

$R^2$ für Halogen, $C_{1-3}$-Halogenalkyl, $S(O)_nCF_3$ oder $SF_5$ steht

n 0, 1 oder 2 bedeutet

$R^4$ für Wasserstoff, Cyano, oder einen Rest der Formel

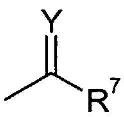

oder einen der folgenden cyclischen Substituenten:

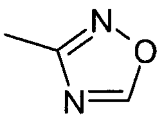

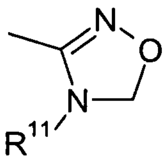

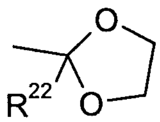

steht

$R^5$ für Wasserstoff, $C_{2-4}$-Alkinyl, $C_{2-4}$-Alkenyl, das gegebenenfalls ein- oder mehrfach substituiert sein kann mit Halogen oder $C_{1-3}$-Alkyl, oder $R^5$ für $C_{1-4}$-Alkyl-(C=O)-, $C_{1-4}$-Alkyl-S-, $C_{1-4}$-Halogenalkyl-S-, -S(=O)-$C_{1-4}$-Alkyl oder -S(=NH)-$C_{1-4}$-Alkyl, gegebenenfalls mit Halogen substituiertes Phenyl, gegebenenfalls mit Halogen substituiertes Furyl, den Rest -$NR^{14}R^{15}$, einen Oxiranylrest, der gegebenenfalls ein- oder mehrfach substituiert ist mit $C_{1-4}$-Alkyl oder $C_{1-4}$-Halogenalkyl, oder einen Cyclopropylrest, der gegebenenfalls ein- oder mehrfach substituiert ist mit Halogen, $C_{1-4}$-Alkyl oder $C_{1-4}$-Halogenalkyl, steht,

$R^6$ für Wasserstoff, $C_{1-4}$-Alkylcarbonyl oder einen Rest -$NR^{16}R^{17}$ steht.

$R^7$ für Wasserstoff, $C_{1-4}$-Alkyl, $C_{1-4}$-Alkyl-S- oder -$NR^9R^{10}$ steht,

Y für =S, =O, =NH, =N-$C_{1-4}$-Alkyl =N-OH, oder

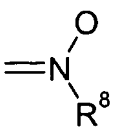

steht,

$R^8$ für $C_{1-4}$-Alkyl steht,

$R^9$ und $R^{10}$ unabhängig voneinander für Wasserstoff, Hydroxy oder $C_{1-4}$-Alkyl stehen,

$R^{11}$ für Wasserstoff, $C_{1-4}$-Alkyl, -COO-$C_{1-4}$-Alkyl oder -$CONR^{12}R^{13}$ steht,

$R^{12}$ und $R^{13}$ unabhängig voneinander für Wasserstoff oder $C_{1-4}$-Alkyl stehen,

$R^{14}$ und $R^{15}$ unabhängig voneinander für Wasserstoff, $C_{1-4}$-Alkyl, $C_{1-4}$-Halogenalkyl oder $C_{1-4}$-Alkyl-$SO_2$- stehen,

$R^{16}$ und $R^{17}$ unabhängig voneinander für Wasserstoff, $C_{1-4}$-Alkoxy oder $C_{1-4}$-Alkyl steht, wobei das $C_{1-4}$-Alkyl gegebenenfalls substituiert sein kann mit Phenyl, Pyranzinyl oder Pyridyl, wobei Phenyl, Pyranzinyl oder Pyridyl ein- oder mehrfach substituiert sein können mit Hydroxy, $C_{1-4}$-Alkyl, $C_{1-4}$-Halogenalkyl und/oder $C_{1-4}$-Alkoxy, oder

$R^{16}$ und $R^{17}$ für $C_{1-4}$-Alkylcarbonyl, $C_{1-4}$-Alkoxycarbonyl, $C_{1-4}$-Alkoxy-$C_{1-4}$-alkylcarbonyl oder den Rest -(C=O)$NR^{20}R^{21}$ steht oder

$R^{16}$ und $R^{17}$ gemeinsam für die durch eine Doppelbindung mit dem Stickstoff verbundene Gruppe =$CR^{18}R^{19}$ stehen,

$R^{18}$ und $R^{19}$ unabhängig voneinander für Phenyl, das gegebenenfalls ein- oder mehrfach substituiert ist mit Hydroxy, $C_{1-4}$-Alkyl, $C_{1-4}$-Halogenalkyl und/oder $C_{1-4}$-Alkoxy, stehen und/oder $R^{18}$ und $R^{19}$ für Wasserstoff, $C_{1-4}$-Alkyl, $C_{1-4}$-Alkenyl oder $C_{1-4}$-Alkoxy stehen, wobei $C_{1-4}$-Alkyl, $C_{1-4}$-Alkenyl oder $C_{1-4}$-Alkoxy gegebenenfalls substituiert sein können mit Phenyl, das gegebenenfalls ein- oder mehrfach substituiert ist mit Hydroxy, $C_{1-4}$-Alkyl, $C_{1-4}$-Halogenalkyl und/oder $C_{1-4}$-Alkoxy-,

$R^{20}$ und $R^{21}$ unabhängig voneinander für Wasserstoff, $C_{1-4}$-Alkyl oder Phenyl, das gegebenenfalls ein- oder mehrfach substituiert ist mit Hydroxy, $C_{1-4}$-Alkyl, $C_{1-4}$-Halogenalkyl und/oder $C_{1-4}$-Alkoxy-, stehen,

$R^{22}$ für $C_{1-4}$-Alkyl steht.

Halogen steht vorzugsweise für Fluor, Chlor, Brom oder Jod, insbesondere Fluor, Chlor Brom.

$C_{1-4}$-Alkyl steht für geradkettiges oder verzweigtes Alkyl mit 1 bis 4 Kohlenstoffatomen, wie z. B. Methyl, Ethyl, n-Propyl, i-Propyl, n-Butyl, sek-Butyl, tert-Butyl.

$C_{1-4}$-Halogenalkyl steht für geradkettiges oder verzweigtes Alkyl mit 1 bis 4 Kohlenstoffatomen, das mit einem oder mehreren gleichen oder verschiedenen

Halogenatomen substituiert ist; dies schließt auch Perhalogenalkylverbindungen ein. Bevorzugt sind Fluoralkyle. Beispiele sind $-CF_2H$, $-CF_3$, $-CH_2CF_3$, $-CF_2CF_3$.

Bevorzugt haben die Substituenten folgende Bedeutungen:

X steht bevorzugt für C-$R^1$.

$R^1$ und $R^3$ stehen bevorzugt unabhängig voneinander für Chlor oder Brom.

$R^2$ steht bevorzugt für $C_{1-3}$-Halogenalkyl oder $SF_5$.

$R^4$ steht bevorzugt für Wasserstoff, Cyano, oder einen Rest der Formel

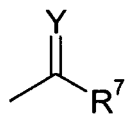

oder einen der folgenden cyclischen Substituenten:

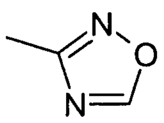 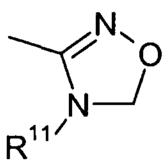 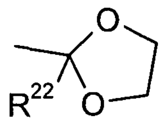

.

$R^5$ steht bevorzugt für Wasserstoff, $C_{2-3}$-Alkinyl, $C_{2-3}$-Alkenyl, das gegebenenfalls einfach substituiert sein kann mit Halogen oder $C_{1-3}$-Alkyl, oder $R^5$ steht bevorzugt für $C_{1-3}$-Alkyl-(C=O)-, $C_{1-3}$-Alkyl-S-, $C_{1-3}$-Halogenalkyl-S-, -S(=O)-$C_{1-3}$-Alkyl oder -S(=NH)-$C_{1-3}$-Alkyl, gegebenenfalls mit Halogen substituiertes Phenyl, gegebenenfalls mit Halogen substituiertes Furyl, den Rest -$NR^{14}R^{15}$, einen gegebenenfalls mit $C_{1-3}$-Halogenalkyl substituierten Oxiranylrest oder einen Cyclopropylrest, der gegebenenfalls ein- oder mehrfach substituiert ist mit Halogen, $C_{1-4}$-Alkyl oder $C_{1-4}$-Halogenalkyl, steht,

$R^6$ steht bevorzugt für Wasserstoff, $C_{1-3}$-Alkylcarbonyl oder einen Rest -$NR^{16}R^{17}$.

$R^7$ steht bevorzugt für Wasserstoff, $C_{1-4}$-Alkyl, $C_{1-4}$-Alkyl-S- oder -$NR^9R^{10}$.

Y steht bevorzugt für =S, =O, =NH, =N-OH, oder

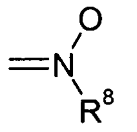

| | |
|---|---|
| $R^8$ | steht bevorzugt für $C_{1-3}$-Alkyl. |
| $R^9$ und $R^{10}$ | stehen bevorzugt unabhängig voneinander für Wasserstoff, Hydroxy oder $C_{1-3}$-Alkyl. |
| $R^{11}$ | steht bevorzugt für Wasserstoff, $C_{1-4}$-Alkyl oder $-CONR^{12}R^{13}$. |
| $R^{12}$ und $R^{13}$ | stehen bevorzugt unabhängig voneinander für Wasserstoff oder $C_{1-3}$-Alkyl. |
| $R^{14}$ und $R^{15}$ | stehen bevorzugt unabhängig voneinander für Wasserstoff, $C_{1-3}$-Alkyl, $C_{1-3}$-Halogenalkyl oder $C_{1-3}$-Alkyl-$SO_2$-. |
| $R^{16}$ und $R^{17}$ | stehen bevorzugt unabhängig voneinander für Wasserstoff, $C_{1-3}$-Alkoxy oder $C_{1-3}$-Alkyl, wobei das $C_{1-3}$-Alkyl gegebenenfalls substituiert sein kann mit Phenyl, Pyrazinyl oder Pyridyl, wobei Phenyl, Pyrazinyl oder Pyridyl ein- oder zweifach substituiert sein können mit Hydroxy, $C_{1-3}$-Alkyl, $C_{1-3}$-Halogenalkyl und/oder $C_{1-3}$-Alkoxy, oder |
| $R^{16}$ und $R^{17}$ | stehen für $C_{1-4}$-Alkylcarbonyl, $C_{1-4}$-Alkoxycarbonyl, $C_{1-4}$-Alkoxy-$C_{1-4}$-alkylcarbonyl oder den Rest $-(C=O)NR^{20}R^{21}$ oder |
| $R^{16}$ und $R^{17}$ | stehen gemeinsam für die durch eine Doppelbindung mit dem Stickstoff verbundene Gruppe $=CR^{18}R^{19}$. |
| $R^{18}$ und $R^{19}$ | stehen bevorzugt unabhängig voneinander für Phenyl, das gegebenenfalls ein- oder zweifach substituiert ist mit Hydroxy, $C_{1-3}$-Alkyl, $C_{1-3}$-Halogenalkyl und/oder $C_{1-3}$-Alkoxy, und/oder $R^{18}$ und $R^{19}$ für Wasserstoff, $C_{1-3}$-Alkyl, $C_{1-3}$-Alkenyl oder $C_{1-3}$-Alkoxy, wobei $C_{1-3}$-Alkyl, $C_{1-3}$-Alkenyl oder $C_{1-3}$-Alkoxy gegebenenfalls substituiert sein können mit Phenyl, das gegebenenfalls ein- oder zweifach substituiert ist mit Hydroxy, $C_{1-4}$-Alkyl, $C_{1-4}$-Halogenalkyl und/oder $C_{1-4}$-Alkoxy-. |
| $R^{20}$ und $R^{21}$ | stehen bevorzugt unabhängig voneinander für $C_{1-3}$-Alkyl oder Phenyl, das gegebenenfalls ein- oder zweifach substituiert ist mit Hydroxy, $C_{1-3}$-Alkyl, $C_{1-3}$-Halogenalkyl und/oder $C_{1-3}$-Alkoxy-. |
| $R^{22}$ | steht bevorzugt für $C_{1-3}$-Alkyl. |

Besonders bevorzugt haben die Substituenten in Formel (I) die folgende Bedeutung:

| | |
|---|---|
| X | steht für C-$R^1$. |
| $R^1$ und $R^3$ | stehen jeweils für Cl. |
| $R^2$ | steht für $CF_3$. |
| $R^4$ | steht für CN, $-C(=S)NH_2$ oder $-C(=O)CH_3$. |
| $R^5$ | steht für $-SCHF_2$, $-S(=O)CF_3$, $-S(=O)CH_3$, $-S(=O)CH_2CH_3$ oder für den 1-Trifluormethyloxiranylrest. |
| $R^6$ | steht für eine Aminogruppe oder einen der folgenden Reste |

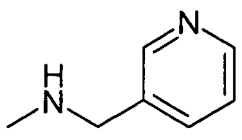

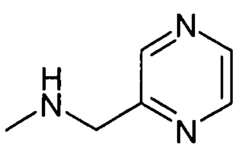

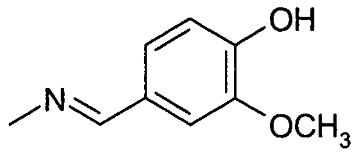

Im Folgenden sind bevorzugte Beispiele erfindungsgemäß verwendbarer Verbindungen angegeben:

EP00511845

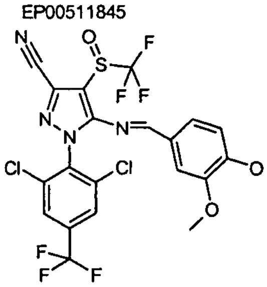

US05321040

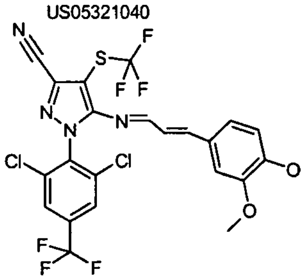

EP00659745

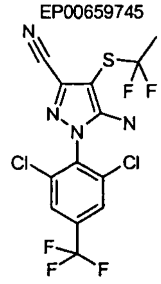

US05556873

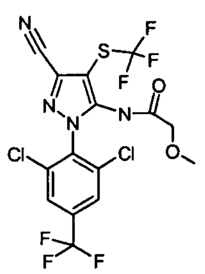

WO09639389

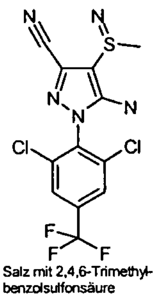

Salz mit 2,4,6-Trimethylbenzolsulfonsäure

US05629335

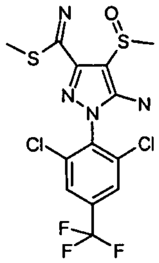

GB02308365

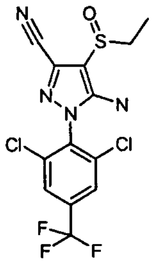

EP00780378

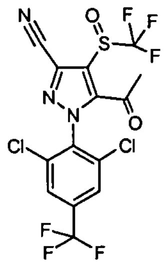

WO09728126

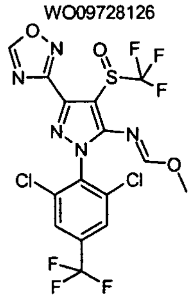

EP00839809

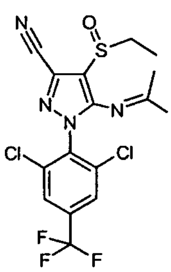

EP00846686

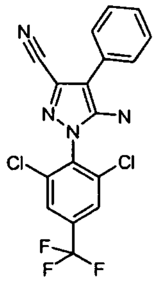

WO09824767

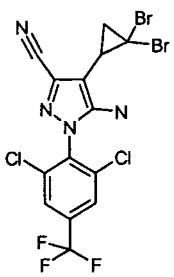

DE19650197

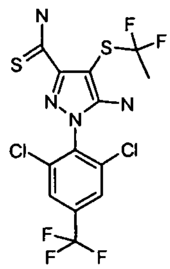

WO09828278

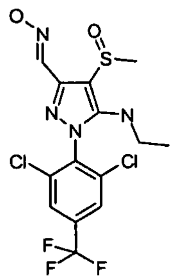

WO09828279

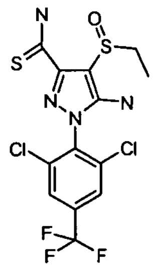

WO09840359

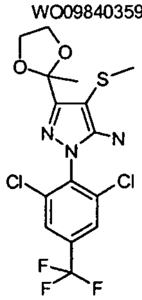

WO09845274

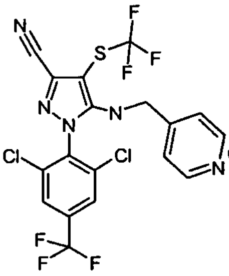

US05814652

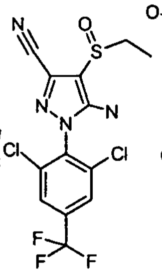

WO09856767

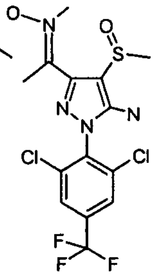

EP00911329

EP00933363

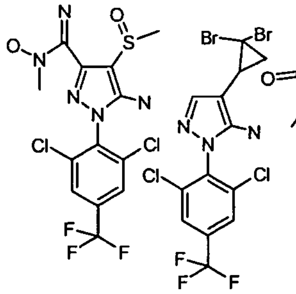

WO09962903

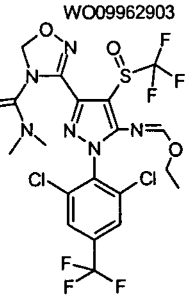

WO09804530

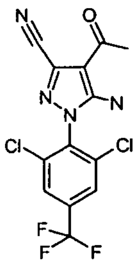

WO200031043, DE19824487

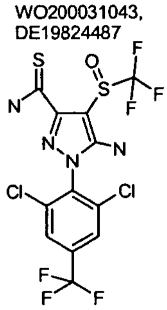

US06069157

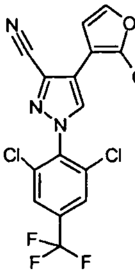

WO09828277

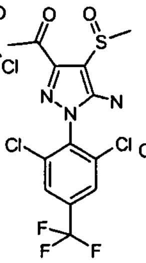

FR2834288 A1 20030704

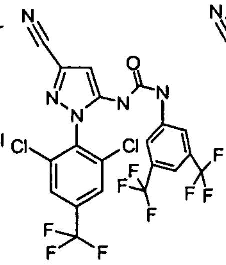

WO2005090313 A1 20050929

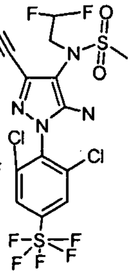

Besonders bevorzugte Beispiele erfindungsgemäß verwendbarer Verbindungen sind:

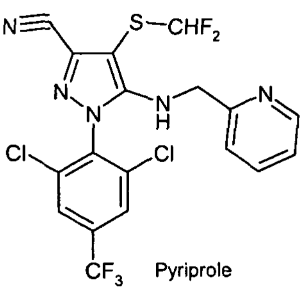
Pyriprole

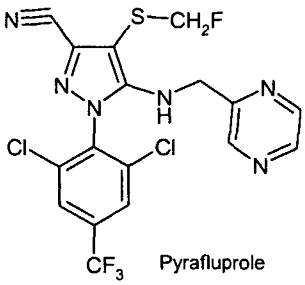
Pyrafluprole

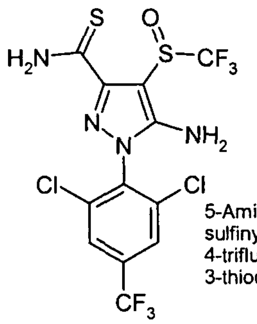
5-Amino-4-trifluormethylsulfinyl-1-(2,6-dichlor-4-trifluormethylphenyl)-3-thiocarbamoylpyrazol

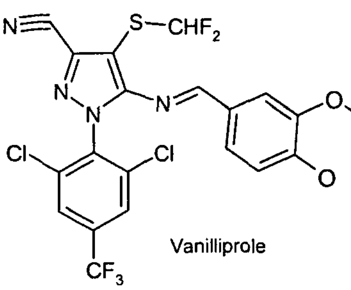
Vanilliprole

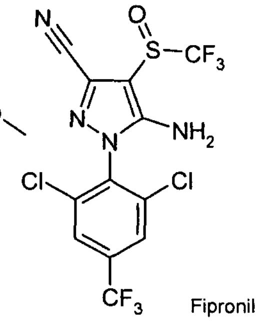
Fipronil

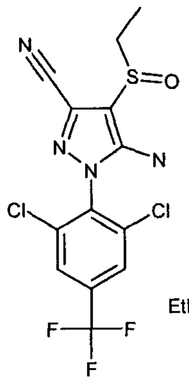
Ethiprole

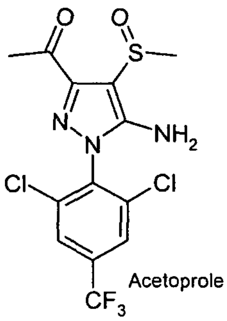
Acetoprole

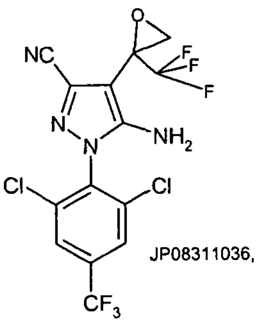
JP08311036, Takeda

Ein Beispiel für ein ganz besonders bevorzugtes N-Arylpyrazol ist Fipronil.

Ein weiteres Beispiel für ein ganz besonders bevorzugtes N-Arylpyrazol ist 5-Amino-4-trifluormethylsulfinyl-1-(2,6-dichlor-4-trifluormethylphenyl)-3-thiocarbamoylpyrazol.

Die Wirkstoffe können gegebenenfalls in Abhängigkeit von Art und Anordnung der Substituenten in verschiedenen stereoisomeren Formen vorliegen, insbesondere als Enantiomere und Racemate. Sowohl die reinen Stereoisomere als auch deren Mischungen können erfindungsgemäß eingesetzt werden.

Gegebenenfalls können die Wirkstoffe auch in Form ihrer Salze eingesetzt werden, wobei pharmazeutisch verwendbare Säureadditionssalze und basische Salze in Frage kommen.

Als pharmazeutisch verwendbare Salze kommen Salze von Mineralsäuren oder organischen Säuren (z. B. Carbonsäuren oder Sulfonsäuren) in Frage. Als Beispiele seien genannt Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure. Als pharmazeutisch verwendbare basische Salze seien zum Beispiel die Alkalisalze, beispielsweise die Natrium- oder Kaliumsalze und die Erdalkalisalze, beispielsweise die Magnesium-, oder Calciumsalze genannt.

Weiterhin können die Wirkstoffe auch in Form ihrer Solvate, insbesondere Hydrate, eingesetzt werden. Unter Solvaten werden sowohl die Solvate, insbesondere Hydrate, der Wirkstoffe selbst als auch die Solvate, insbesondere Hydrate, von deren Salzen verstanden.

Die Wirkstoffe können als Feststoff unter Umständen verschiedene Kristallmodifikationen bilden. Vorteilhaft für die Anwendung in Arzneimitteln sind stabile Modifikationen, die geeignete Löslichkeitseigenschaften haben.

Soweit nicht anders angegeben sind Prozentangaben als Gewichtsprozente bezogen auf das Gewicht der

fertigen Zubereitung zu verstehen.

Üblicherweise enthalten die Mittel Arylpyrazol in Mengen 1 bis 27,5 Gew.-%, vorzugsweise 5 bis 20 Gew.-%, besonders bevorzugt 7,5 bis 15 Gew.-%.

Das aliphatische, cyclische Carbonat ist vorzugsweise Ethylen- oder Propylencarbonat, wobei auch Mischungen eingesetzt werden können.

Die Menge an aliphatischem cyclischem Carbonat in der Formulierung kann im Bereich 10 Gew.-% bis 70 Gew.-% , bevorzugt 12,5 bis 50 Gew-%, besonders bevorzugt 15 bis 40 Gew-% breit variiert werden.

Aliphatische cyclische oder acyclische Polyether sind an sich bekannte Verbindungen. Bevorzugt handelt es sich um Ether, die sich von Diolen mit bis zu 8 Kohlenstoffatomen, wie z. B. Ethylenglykol, Diethylenglykol, Propylenglykol, Dipropylenglykol ableiten. In den acyclischen Ethern tragen ein oder beide OH-Gruppen eine $C_{1-4}$-Alkylgruppe, bevorzugt ist nur eine OH-Gruppe verethert; besonders bevorzugte Beispiele sind: Diethylenglykolmonoethylether, Diethylenglykolmonopropylether Dipropylenglykolmonopropylether. Bevorzugte 5- oder 6-gliedrige cyclische Ether haben einen Ringsauerstoff und 4 oder 5 Ringkohlenstoffatome und tragen gegebenenfalls eine $C_{1-4}$-Alkylsubstituenten; bevorzugt tragen sie eine freie OH-Gruppe entweder direkt am Ring oder an dem $C_{1-4}$-Alkylsubstituenten. Die Menge an aliphatischem, cyclischem oder acyclischem Polyether in den erfindungsgemäßen Mitteln kann von 20 bis 77,5 Gew-% breit variiert werden, wobei die Mengen im Bereich 25 bis 65 Gew- % besonders und die Mengen im Bereich 25 bis 50 Gew-% ganz besonders zu bevorzugen sind.

Die erfindungsgemäßen Mittel enthalten zusätzlich einen oder mehrere Ester eines zwei- oder dreiwertigen Alkohols mit bis zu drei Kohlenstoffatomen mit organischen Fettsauren mit 6 bis 18 Kohlenstoffatomen. Die erfindungsgemäß eingesetzten Ester enthalten als Alkoholkomponente einen zwei oder dreiwertigen Alkohole mit bis zu drei Kohlenstoffatomen, wie z. B. Ethylenglykol, Propylenglykol oder Glycerin. In der Regel sind mindestens zwei, vorzugsweise alle Hydroxylgruppen des Alkohols verestert. Säurekomponente der Ester sind Fettsäuren mit 6 bis 18 Kohlenstoffatomen, die geradkettig, verzweigt sowie ein- oder mehrfach ungesättigt sein können. Es können gemischte Ester oder auch Mischungen verschiedener Estertypen eingesetzt werden. Als Triglyceride bevorzugt sind Capryl-Caprinsäure-Triglyceride sowie Capryl-Caprin-Linolsäure-Triglyceride. In gleicher Weise bevorzugt sind Ester des Propylenglykols mit Capryl- und/oder Caprinsäure (Propylenglykoloctanoatdecanoat). Besonders bevorzugt weisen diese Glycerin- bzw. Propylenglykolester der Capryl-/Caprinsäure einen Viskositätsbereich (20°C) von 0,08 - 1,3 Pa.s, vorzugsweise jedoch 0,08 - 0,40 Pa.s auf. Ebenfalls eingesetzt werden können deren Polyethylenoxid-, Polypropylenoxid- und/oder Propylencarbonat-modifizierte Derivate mit dem genannten Viskositätsbereich. Als Beispiele seien Propylenglykoldicaprylat, Propylenglykoloctanoatdecanoat im Viskositätbereich 0,09-0,12 Pa.s, Capryl-Caprin-Diglyceryl-Succinat mit mittlerer Viskosität von 0,23 Pa.s, mittelkettige Capryl-Caprin-Triglyceride mit der Viskosität 0,27 - 0,30 Pa.s erwähnt.

Die erfindungsgemäßen Flüssigformulierungen können einen oder mehrere der o.g. Ester enthalten. Üblicherweise enthalten die erfindungsgemäßen Mittel den Ester oder das Estergemisch in Anteilen von 0 bis 40 Gew.-%, bevorzugt 1 bis 35 Gew.-%, besonders bevorzugt 1 bis 12,5 Gew-% und ganz besonders bevorzugt 2.5 bis 7.5 Gew-%.

Zur Stabilisierung der genannten Formulierungen können gegebenenfalls übliche organische oder anorganische Antioxidantien eingesetzt werden. Als anorganische Antioxidantien seien z. B. die Sulfite und Bisulfite, insbesondere Natriumbisulfit genannt. Bevorzugt sind phenolische Antioxidantien wie Anisol, Butylhydroxytoluol und Hydroxyanisol bzw. deren Mischungen untereinander. Üblicherweise werden 0,01 bis 1 Gew- %, bevorzugt 0.05% bis 0.5%, besonders bevorzugt 0.075 bis 0.2 Gew.-% eingesetzt.

Die genannten Formulierung-Einsatzstoffe, insbesondere die organischen Ester können mittels Säuerungsmitteln gegen einen möglichen hydrolytischen Abbau stabilisiert werden. Als Säuerungsmittel eignen sich pharmazeutisch annehmbare Säuren, insbesondere Carbonsäuren, wie z. B. Bernstein-, Wein- Milch- oder Zitronensäure Ihre bevorzugte Menge liegt im Bereich 0 bis 0,5 Gew.%, vorzugsweise jedoch 0 bis 0,2 Gew- %.

Als weitere Formulierhilfsmittel können zur Verbesserung der Spreitwirkung polymere Tenside auf der Basis von Polymethoxysiloxanen mit geringer Oberflächenspannung < 30 mN/m vorzugsweise < 22 mN/m eingesetzt werden. Derartige Tenside sind bekannte ethoxylierte und/oder propoxylierte, vorzugsweise neutrale oder besonders bevorzugt kationische Formulierhilfsmittel. Als Beispiel für ein bevorzugtes polymeres Hilfsmittel sei auf das Methoxysilanethylenoxidcopolymerisat Belisil Silvet L 77 der Fa. Bayer GE Siliconics GmbH hingewiesen. Die Menge an diesen Formulierhilfsmitteln kann im Bereich 0,01 bis 1,0 Gew.-% breit variiert werden. Der bevorzugte Bereich liegt bei 0,2 bis 0,4 Gew.-%.

Die Formulierungen können gegebenenfalls weitere pharmazeutisch annehmbare Hilfs- und Zusatzstoffe enthalten.

Die erfindungsgemäßen Mittel können auch einen oder mehrere zusätzliche Wirkstoffe neben den Arylpyrazolen und Pyrethroiden enthalten. Als bevorzugte Beispiele für solche Kombinationswirkstoffe seien genannt: Wachstumshemmer, wie z.B. Chitinbiosynthesehemmer wie z.B. Benzoylphenylharnstoffe (z.B. Triflumuron, Lufenuron); Phenyloxazoline (z. B. Etoxazol); Juvenilhormonanaloga (z. B. Methopren, Hydropren, Pyriproxifen) sowie Mischungen dieser Wirkstoffe untereinander genannt. Ihre Menge kann im Bereich 0,1 bis 7,5 Gew. % vorzugsweise jedoch 0,25 bis 5,0 Gew. %, besonders bevorzugt 0,25 bis 2,5 Gew. % breit variiert werden.

Die erfindungsgemäßen Formulierungen können auch Synergisten enthalten. Als Synergisten im Sinne dieser

Anmeldung werden Verbindungen verstanden, die selbst nicht die gewünschte Wirksamkeit aufweisen, als Mischpartner jedoch zu einer Steigerung der Wirksamkeit der aktiven Wirkstoffe führen. Beispielhaft seien hier genannt Piperonylbutoxid, MGK264, Verbutin, S,S,S-Tributylphosphorotrithioat. Synergisten werden in den erfindungsgemäßen Formulierungen bevorzugt bei α-Cyanopyrethroiden eingesetzt, und zwar in einem Synergist:Pyrethroid-Verhältnis von 20-50 : 1 (siehe auch WO 04/098290). Bevorzugter Synergist ist MGK264.

Die erfindungsgemäßen Mittel sind umweltverträglich und weisen eine geringe gegenüber bekannten Mitteln reduzierte Toxizität auf. Sie sind daher anwenderfreundlich und zeichnen sich zudem durch ihre einfache Handhabung aus. Die Mittel haben einen günstigen Flammpunkt von > 70°C und können daher in einfachen Anlagen, die keine zusätzlichen Explosionsschutzmassnahmen erfordern, hergestellt werden.

Die erfindungsgemäßen Mittel eignen sich bei günstiger Warmblütertoxizität zur Bekämpfung von parasitierenden Arthropoden, insbesondere Insekten und Spinnentiere, ganz besonders Flöhen und Zecken, die bei Tieren, insbesondere bei Warmblütern, besonders bevorzugt bei Säugetieren vorkommen. Dies können Haus- und Nutztiere sowie Zoo-, Labor-, Versuchs- und Hobbytiere sein.

Die hier beschriebenen Mittel werden insbesondere gegen Ektoparasiten an Hobbytieren insbesondere Hunde und Katzen und Nutztieren eingesetzt.

Die erfindungsgemäßen Mittel sind dabei gegen alle oder einzelne Entwicklungsstadien der Schädlinge sowie gegen resistente und normal sensible Arten der Schädlinge wirksam.

Zu den Schädlingen gehören:

Aus der Ordnung der Anoplura z.B. Haematopinus spp., Linognathus spp., Solenopotes spp., Pediculus spp., Pthirus spp.;

aus der Ordnung der Mallophaga z.B. Trimenopon spp., Menopon spp., Eomenacanthus spp., Menacanthus spp., Trichodectes spp., Felicola spp., Damalinea spp., Bovicola spp;

aus der Ordnung der Diptera in der Unterordnung Brachycera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp..

aus der Ordnung der Diptera in der Unterordnung Nematocera z.B. Culex spp., Aedes spp., Anopheles spp., Culicoides spp., Phlebotomus spp., Simulium spp..

aus der Ordnung der Siphonaptera z.B. Ctenocephalides spp., Echidnophaga spp., Ceratophyllus spp., Pulex spp..

aus der Ordnung der Metastigmata z.B. Hyalomma spp., Rhipicephalus spp., Boophilus spp., Amblyomma spp., Haemaphysalis spp., Dermacentor spp., Ixodes spp., Argas spp., Ornithodorus spp., Otobius spp.;

aus der Ordnung der Mesostigmata z.B. Dermanyssus spp., Ornithonyssus spp., Pneumonyssus spp..

aus der Ordnung der Prostigmata z.B. Cheyletiella spp., Psorergates spp., Myobia spp., Demodex spp., Neotrombicula spp.;

aus der Ordnung der Astigmata z.B. Acarus spp., Myocoptes spp., Psoroptes spp., Chorioptes spp., Otodectes spp., Sarcoptes spp., Notoedres spp., Knemidocoptes spp., Neoknemidocoptes spp. Cytodites spp., Laminosioptes spp..

Besonders hervorgehoben sei die Wirkung gegen Flöhe (Siphonaptera z.B. Ctenocephalides spp., Echidnophaga spp., Ceratophyllus spp., Pulex spp.), Zecken (Hyalomma spp., Rhipicephalus spp., Boophilus spp., Amblyomma spp., Haemaphysalis spp., Dermacentor spp., Ixodes spp., Argas spp., Ornithodorus spp., Otobius spp.) und die oben genannten Dipteren (Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp.).

Zu den Nutz und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten.

Zu Labor und Versuchstieren gehören Mäuse, Ratten, Meerschweinchen, Kaninchen, Goldhamster, Hunde und Katzen.

Zu den Hobbytieren gehören Hunde und Katzen.

Insbesondere hervorgehoben sei die Anwendung bei Katze und Hund.

Die Anwendung kann sowohl prophylaktisch als auch therapeutisch erfolgen.

Die erfindungsgemäßen Flüssigformulierungen sind vorzugsweise für die Spot on, Pour on oder Spray-Applikation geeignet, wobei die Spray-Applikation z. B. mit einem Pumpspray oder einem Aerosolspray (Druckspray) erfolgen kann. Für spezielle Indikationen ist auch die Verwendung nach Verdünnung mit Wasser als Tauchbad denkbar; in diesem Fall sollte die Formulierung emulgierende Zusätze enthalten.

Die bevorzugten Applikationsformen sind Pump Spray, Pour on und Spot on. Die Spot on Applikation ist ganz besonders bevorzugt.

Die erfindungsgemäßen Formulierungen zeichnen sich durch ihre hervorragende Kompatibilität mit den herkömmlichen "Single-Dose" Kunststofftuben und durch ihre Lagerstabilität in verschiedenen Klimazonen aus. Sie sind niedrigviskos und problemlos applizierbar.

Die erfindungsgemäßen, flüssigen Formulierungen können hergestellt werden, indem man Bestandteile in entsprechenden Mengen miteinander vermischt, z.B. durch den Einsatz konventioneller Rührkessel oder anderer geeigneter Geräte. Falls die Inhaltsstoffe es erfordern, kann auch unter Schutzatmosphäre oder mit anderen Methoden des Sauerstoffausschlusses gearbeitet werden.

Beispiele:

Beispiel 1

| | |
|---|---|
| 100 ml | Flüssigformulierung bestehend aus |
| 10,0 g | 5-Amino-4-trifluormethylsulfinyl-1-(2,6-dichlor-4-trifluormethylphenyl)-3-thiocarbamoylpyrazol |
| 57,30 g | Diethylenglykolmonoethylether |
| 0.10 g | BHT |
| 0,20 g | BHA |
| 30,02 g | Propylencarbonat |
| 5,00 g | Propylenglykoloctanoatdecanoat |
| 0,24 g | Flumethrin |
| 10,36 g | MGK 264 |
| 0,02 g | Zitronensäure |

Beispiel 2

| | |
|---|---|
| 100 ml | Flüssigformulierung bestehend aus |
| 10,00 g | 5-Amino-4-trifluormethylsulfinyl-1-(2,6-dichlor-4-trifluormethylphenyl)-3-thiocarbamoylpyrazol |
| 0.24 g | Flumethrin |
| 0,02 g | Zitronensäure |
| 0,20 g | BHT |
| 68,00 g | Dipropylenglykolmonomethylether |
| 13,40 g | Propylencarbonat |
| 5,00 g | Wasser entm. |
| 5,00 g | Propylenglykoloctanoatdecanoat |
| 5,00 g | MGK 264 |

Beispiel 3

| | |
|---|---|
| 100 ml | Flüssigformulierung bestehend aus |
| 10,00 g | 5-Amino-4-trifluormethylsulfinyl-1-(2,6-dichlor-4-trifluormethylphenyl)-3-thiocarbamoylpyrazol |
| 0,50 g | PPF (Pyriproxyfen) |

| | |
|---|---|
| 0.24 g | Flumethrin |
| 0,02 g | Zitronensäure |
| 0,20 g | BHT |
| 67,50 g | Dipropylenglykolmonomethylether |
| 13,40 g | Propylencarbonat |
| 5,00 g | Wasser entm. |
| 5,00 g | Propylenglykoloctanoatdecanoat |
| 5,00 g | MGK 264 |

Beispiel 4

| | |
|---|---|
| 100 ml | Flüssigformulierung bestehend aus |
| 10,00 g | 5-Amino-4-trifluormethylsulfinyl-1-(2,6-dichlor-4-trifluormethylphenyl)-3-thiocarbamoylpyrazol |
| 0,50 g | PPF (Pyriproxyfen) |
| 0.24 g | Flumethrin |
| 0,02 g | Zitronensäure |
| 0,20 g | BHT |
| 60,90 g | Diethylenglykolmonoethylether |
| 20,00 g | Propylencarbonat |
| 5,00 g | Wasser entm. |
| 5,00 g | Propylenglykoloctanoatdecanoat |
| 5,00 g | MGK 264 |

Beispiel 5

| | |
|---|---|
| 100 ml | Flüssigformulierung bestehend aus |
| 10,0 g | 5-Amino-4-trifluormethylsulfinyl-1-(2,6-dichlor-4-trifluormethylphenyl)-3-thiocarbamoylpyrazol |
| 45,00 g | Permethrin |
| 37,90 g | Diethylenglykolmonoethylether |
| 0.10 g | BHT |
| 0,20 g | BHA |
| 25,00 g | Propylencarbonat |
| 5,00 g | Propylenglykoloctanoatdecanoat |
| 0,02 g | Zitronensäure |

Beispiel 6

| | |
|---|---|
| 100 ml | Flüssigformulierung bestehend aus |
| 10,0 g | 5-Amino-4-trifluormethylsulfinyl-1-(2,6-dichlor-4-trifluormethylphenyl)-3-thiocarbamoylpyrazol |
| 45,00 g | Permethrin |
| 1,00 g | PPF |
| 36,90 g | Diethylenglykolmonoethylether |
| 0.10 g | BHT |
| 0,20 g | BHA |
| 25,00 g | Propylencarbonat |
| 5,00 g | Propylenglykoloctanoatdecanoat |
| 0,02 g | Zitronensäure |

Beispiel 7

| | |
|---|---|
| 100 ml | Flüssigformulierung bestehend aus |
| 10,0 g | 5-Amino-4-trifluormethylsulfmyl-1-(2,6-dichlor-4-trifluormethylphenyl)-3-thiocarbamoylpyrazol |
| 45,00 g | Permethrin |
| 1,00 g | PPF |
| 0.25 g | Silvet L 77 der Fa. GE Siliconocs GmbH D-51368 Leverkusen |
| 36,65 g | Diethylenglykolmonoethylether |
| 0.10 g | BHT |
| 0,20 g | BHA |
| 25,00 g | Propylencarbonat |
| 5,00 g | Propylenglykoloctanoatdecanoat |
| 0,02 g | Zitronensäure |

Beispiel 8

| | |
|---|---|
| 10,0 g | 5-Amino-4-trifluormethylsulfinyl-1-(2,6-dichlor-4-trifluormethylphenyl)-3-thiocarbamoylpyrazol |
| 45,00 g | Permethrin |
| 1,00 g | PPF |
| 0.25 g | Silvet L 77 (Fa. Bayer-GE Silicones GmbH, D-51368 Leverkusen) |
| 36,65 g | Diethylenglykolmonoethylether |
| 0.10 g | BHT |
| 0,20 g | BHA |
| 25,00 g | Ethylencarbonat |
| 5,00 g | Propylenglykoloctanoatdecanoat |
| 0,02 g | Zitronensäure |

Vergleichsbeispiel 1

Eine im Handel erhältliche, Fipronil 10 % Spot on Formulierung der Fa. Merial Ltd., 3239 Satellite Blvd., Duluth, GA 30096-4640, USA.

Vergleichsbeispiel 2:

Eine Formulierung enthaltend 5-Amino-4-trifluormethylsulfinyl-1-(2,6-dichlor-4-trifluormethylphenyl)-3-thiocarbamoylpyrazol jedoch ohne Zusatz von Flumethrin und MGK264:

| | |
|---|---|
| 100 ml | Flüssigformulierung bestehend aus: |
| 10,0 g | 5-Amino-4-trifluormethylsulfinyl-1-(2,6-dichlor-4-trifluormethylphenyl)-3-thiocarbamoylpyrazol |
| 57,7 g | Diethylenglykolmonoethylether |
| 40,0 g | Propylencarbonat |
| 5,0 g | Propylenglykoloctanoatdecanoat |
| 0,1 g | Butylhydroxytoluol |
| 0,2 g | Butlyhydroxyanisol |

Vergleichsbeispiel 3:

Eine Formulierung enthaltend Flumethrin und MGK264 und PPF jedoch statt des in der Anmeldung genannten 3-Thiocarbamoylpyrazols das bekannte Insektizid Imidacloprid.

| | |
|---|---|
| 100 ml | Flüssigformulierung bestehend aus |
| 10,0 g | Imidacloprid |
| 0,50 g | PPF |
| 56,80 g | Benzylalkohol |
| 0.10 g | BHT |
| 0,20 g | BHA |
| 30,02 g | Propylencarbonat |
| 5,00 g | Propylenglykoloctanoatdecanoat |

| | |
|---|---|
| 0,24 g | Flumethrin |
| 10,36 g | MGK 264 |
| 0,02 g | Zitronensäure |

**Biologische Beispiele**

Alle Verbindungen wurden nach Gewicht exakt dosiert, um bessere Vergleichbarkeit zu gewährleisten. Dazu wurden 20 Pipetten des Fipronil-haltigen Handelpräparates in eine Glasflasche entleert und ebenfalls über einen Code verblindet.
Alle Proben wurden mit Eppendorfpipetten (bis 0,95 ml Volumen) als einzelner Spot am Nacken aufgetragen (Katzen und kleinere Hunde). Bei Applikationsvolumina über 1 ml wurde das Volumen halbiert und in zwei Spots im Abstand von ca. 10 cm am Nacken aufgetragen.
Aus den weiteren Laborprüfungen zur Floh- und Zecken-Wirksamkeit gemäß Beispiel 2 geht hervor, dass die Präparate in den o.a. erfindungsgemäßen Formulierungen eine sehr gute und langanhaltende Wirkung gegen Zecken und Flöhe aufweisen die in den Prüfungen durchweg über dem Stand der Technik (VB 1- VB 3) liegt. Weiter zeichnen sich die Präparate in den o.a. erfindungsgemäßen Formulierungen durch ihre Zieltier und Anwenderverträglichkeit aus und eignen sich somit hervorragend zum Bekämpfen von Flöhen und Zecken an Kleintieren.

A. Wirksamkeit gegen Flöhe (Ctenocephalides felis) am Hund

Zwischen Tag -4 und -1 werden Hunde 1-2 mal mit ca. 100 adulten, nüchternen Ctenocephalides felis pro Hund infestiert. Dabei werden die Flöhe auf den Nacken des Tieres ausgebracht.
Am Tag 0 wird der Infestationserfolg am Hund überprüft, indem am wachen Tier nach Flöhen gesucht wird. Die Zahl der lebenden Flöhe wird protokolliert.
Nach der Zählung der Flöhe werden die Tiere behandelt. Die Hunde der Kontrollgruppe werden nicht behandelt. Die zu prüfenden Arzneimittel werden den Tieren dermal als Spot-on bei einer Applikationsmenge von 0,1-0.15 ml/kg Körpergewicht oder als Spray mit einer Applikationsmenge von 1-1.5 ml/kg Körpergewicht verabreicht. Die Applikation erfolgt einmalig am Tag 0. Es werden nur klinisch gesunde Tiere verwendet.
Am Tag 1 und 2 werden alle Hunde auf lebende Flöhe überprüft. Die Ergebnisse werden in den Rohdaten festgehalten.
Am Tag 7, 14, 21, 28, 35, gegebenenfalls auch noch an Tag 42 und 49 werden alle Hunde mit ca. 100 adulten, nüchternen Ctenocephalides felis pro Hund reinfestiert. Jeweils einen Tag nach Reinfestation werden alle Hunde auf lebende Flöhe kontrolliert. Die Ergebnisse werden in den Rohdaten protokolliert.
Eine Formulierung wird als hochwirksam erachtet, wenn zwischen 24 und 48 Stunden nach Reinfestation eine Wirksamkeit >95% festgestellt wird und diese Wirkung über mindestens 3-4 Wochen anhält.
Für die Berechnung der Wirksamkeit wird eine modifizierte Formel nach Abbott benutzt:

$$\text{Wirksamkeit \%} = \frac{\textbf{Ø Anzahl Flöhe KG} - \textbf{Ø Anzahl Flöhe BG}}{\textbf{Ø Anzahl Flöhe KG}} \times 100$$

KG: Kontrollgruppe; BG: Behandlungsgruppe
Die Arzneimittel gemäß den Formulierungsbeispiel 2 in einer Dosierung von. 0.15 ml/kg als Spot on appliziert, erwiesen sich gegen Ctenocephalides felis als hochwirksam.

B. Wirksamkeit gegen Zecken (Rhipicephalus sanguineus, Dermacentor variabilis) am Hund

Zwischen Tag -4 und -1 werden Hunde mit 2% Rompun®□ (Bayer AG, Wirkstoff: Xylazinhydrochlorid) (0,1ml/kg Körpergewicht) sediert. Nachdem alle Hunde sediert sind (nach ca. 10-15 Minuten) werden sie in Transportboxen überführt und 50 Rhipicephalus sanguineus oder Dermacentor variabilis (25♀, 25♂) pro Hund auf den Nacken des Tieres ausgebracht. Die Tiere werden nach ca. 1 ½. Stunden wieder aus der Transportkiste in den Käfig gesetzt.
Am Tag 0 wird der Infestationserfolg am Hund überprüft, indem am wachen Tier nach Zecken gesucht wird. Intensiv wird dabei gesucht im Kopf- und Ohrenbereich inkl. Ohrenfalte, im Bereich des Nackens, am Unterbauch, an der Unterbrust, an der seitlichen Flanke sowie zwischen den Zehen und an den Gliedmaßen. Die Zahl der angesogenen lebenden Zecken wird protokolliert. Tote Zecken werden entfernt.
Nach der Zählung der Zecken werden die Tiere behandelt. Die Hunde der Kontrollgruppe werden nicht behandelt. Die zu prüfenden Arzneimittel werden den Tieren dermal als Spot-on mit 0,1-0.15 ml/kg Körpergewicht oder als Spray mit 1-1.5 ml/kg Körpergewicht verabreicht. Die Applikation erfolgt einmalig am Tag 0. Es werden nur klinisch gesunde Tiere verwendet.

Am Tag 1 und Tag 2 werden alle Hunde auf lebende und tote angesogende Zecken überprüft. Die Ergebnisse werden in den Rohdaten festgehalten. Am Tag 2 werden alle lebenden und toten Zecken vom Hund entfernt.
Am Tag 7, 14, 21, 28, 35, gegebenenfalls auch noch an Tag 42 und 49 werden alle Hunde mit jeweils 50 Rhipicephalus sanguineus oder Dermacentor variabilis (25♀, 25♂) pro Hund reinfestiert. Jeweils zwei Tage nach Reinfestation werden alle Hunde auf lebende und tote angesogene Zecken kontrolliert. Die Ergebnisse werden in den Rohdaten protokolliert. Am zweiten Tag nach Reinfestation werden alle lebenden und toten Zecken vom Hund entfernt. Eine Formulierung wird als hochwirksam erachtet, wenn am Tag 2 und jeweils am zweiten Tag nach Reinfestation eine Wirksamkeit >90 % festgestellt wird und diese Wirkung über mindestens 3 Wochen anhält.
Für die Berechnung der Wirksamkeit wird eine modifizierte Formel nach Abbott benutzt:

$$\text{Wirksamkeit \%} = \frac{\text{Ø Anzahl Zecken KG} - \text{Ø Anzahl Zecken BG}}{\text{Ø Anzahl Zecken KG}} \times 100$$

KG: Kontrollgruppe; BG: Behandlungsgruppe

Die Arzneimittel in einer Dosierung gemäß den Formulierungsbeispiel 2 von 0,15 ml/kg als Spot on appliziert, erwiesen sich gegen Rhipicephalus sanguineus als hochwirksam.

C. Wirksamkeit gegen Flöhe (Ctenocephalides felis) an der Katze

An Tag -1 werden Katzen mit ca. 100 adulten, nüchternen Ctenocephalides felis pro Katze infestiert. Dabei werden die Flöhe auf den Nacken des Tieres ausgebracht.
Am Tag 0 wird der Infestationserfolg an der Katze überprüft, indem am wachen Tier nach Flöhen gesucht wird. Die Zahl der lebenden Flöhe wird protokolliert.
Nach der Zählung der Flöhe werden die Tiere behandelt. Die Katzen der Kontrollgruppe werden nicht behandelt. Die zu prüfenden Arzneimittel werden den Tieren dermal als Spot-on bei einer Applikationsmenge von 0,1-0,15 ml/kg Körpergewicht verabreicht. Die Applikation erfolgt einmalig am Tag 0. Es werden nur klinisch gesunde Tiere verwendet. An Tag 2 werden alle Katzen auf lebende Flöhe überprüft. Die Ergebnisse werden in den Rohdaten festgehalten.
Am Tag 7, 14, 21, 28, 35, gegebenenfalls auch noch an Tag 42 und 49 werden alle Katzen mit ca. 100 adulten, nüchternen Ctenocephalides felis pro Katze reinfestiert. Jeweils zwei Tage nach Reinfestation werden alle Katzen auf lebende Flöhe kontrolliert. Die Ergebnisse werden in den Rohdaten protokolliert.
Eine Formulierung wird als hochwirksam erachtet, wenn am Tag 2 und jeweils am zweiten Tag nach Reinfestation eine Wirksamkeit >95% festgestellt wird und diese Wirkung über mindestens 3-4 Wochen anhält.
Für die Berechnung der Wirksamkeit wird eine modifizierte Formel nach Abbott benutzt:

$$\text{Wirksamkeit \%} = \frac{\text{Ø Anzahl Flöhe KG} - \text{Ø Anzahl Flöhe BG}}{\text{Ø Anzahl Flöhe KG}} \times 100$$

KG: Kontrollgruppe; BG: Behandlungsgruppe
Die Arzneimittel gemäß den Formulierungsbeispiel 2 in einer Dosierung von 0,15 ml/kg als Spot on appliziert, erwiesen sich gegen Ctenocephalides felis als hochwirksam.

D. Wirksamkeit gegen Zecken (Ixodes ricinus) an der Katze

Jeweils am Tag -2 werden Katzen mit einem milden Sedativum (Acepromazin maleat) sediert. Nachdem alle Katzen sediert sind (nach ca. 10-15 Minuten) werden 30-50 Ixodes ricinus (15-25♀, 15-25♂) pro Katze auf den Nacken des Tieres ausgebracht.
Am Tag -1 wird der Infestationserfolg an den Katzen überprüft, indem am wachen Tier nach Zecken gesucht wird. Intensiv wird dabei gesucht im Kopf- und Ohrenbereich, im Bereich des Nackens, am Unterbauch, an der Unterbrust, an der seitlichen Flanke sowie an den Gliedmaßen. Die Zahl der angesogenen lebenden Zecken wird protokolliert. Tote Zecken werden entfernt.
Nach der Zählung der Zecken werden die Tiere gruppiert. Die Behandlung erfolgt an Tag 0. Die Katzen der Kontrollgruppe werden nicht behandelt. Die zu prüfenden Arzneimittel werden den Tieren dermal als Spot-on mit 0,1-0,15 ml/kg Körpergewicht verabreicht. Die Applikation erfolgt einmalig am Tag 0. Es werden nur klinisch gesunde Tiere verwendet. An Tag 2 werden alle Katzen auf lebende und tote angesogende Zecken überprüft. Die Ergebnisse werden in den Rohdaten festgehalten. Alle lebenden und toten Zecken werden von der Katze entfernt.
Am Tag 7, 14, 21, 28, 35, gegebenenfalls auch noch an Tag 42 und 49 werden alle Katzen mit jeweils 30-50 Ixodes

ricinus (15-25♀, 15-25♂) pro Katze reinfestiert. Jeweils zwei Tage nach Reinfestation werden alle Katzen auf lebende und tote angesogene Zecken kontrolliert. Die Ergebnisse werden in den Rohdaten protokolliert. Am zweiten Tag nach Reinfestation werden alle lebenden und toten Zecken von der Katze entfernt.
Eine Formulierung wird als hochwirksam erachtet, wenn am Tag 2 und jeweils am zweiten Tag nach Reinfestation eine Wirksamkeit >90 % festgestellt wird und diese Wirkung über mindestens 3 Wochen anhält.
Für die Berechnung der Wirksamkeit wird eine modifizierte Formel nach Abbott benutzt:

$$\text{Wirksamkeit \%} = \frac{\text{Ø Anzahl Zecken KG} - \text{Ø Anzahl Zecken BG}}{\text{Ø Anzahl Zecken KG}} \times 100$$

KG: Kontrollgruppe; BG: Behandlungsgruppe
Die Arzneimittel in einer Dosierung gemäß den Formulierungsbeispiel 2 von 0,15 ml/kg als Spot on appliziert, erwiesen sich gegen Ixodes ricinus als hochwirksam.

E. Floh- und Zeckenwirksamkeit über 4 bis 7 Wochen

Die Floh- und Zeckenwirksamkeit der erfindungsgemäßen Mittel wurde über vier bis sieben Wochen getestet. Die Versuchsdurchführung folgte der Beschreibung unter den Punkten A bis D.

**Tabelle 1a** Flohwirksamkeit des Mittels gemäß Beispiel 2 an der Katze

| | T0 | Appl. Vol | Parasit | W0 | | W1 | | W2 | | W3 | | W4 | | W5 |
|---|---|---|---|---|---|---|---|---|---|---|---|---|---|---|
| 1.Infestation Tag -4 | Behandlung | ml/kg | | T2 | 2.Infestation Tag 7 | T9 | 3.Infestation Tag 14 | T16 | 4.Infestation Tag 21 | T2 3 | 5.Infestation Tag 28 | T30 | 6.Infestation Tag 35 | T37 |
| | VB 1 | 0.1 | Ctenocephalides felis | 97 | | 100 | | 100 | | 100 | | 99 | | 100 |
| | VB 2 | 0.15 | Ctenocephalides felis | 99 | | 100 | | 100 | | 100 | | 100 | | 99 |
| | Beispiel 2 | 0.15 | Ctenocephalides felis | 99 | | 100 | | 100 | | 100 | | 100 | | 100 |
| | VB 3 | 0.1 | Ctenocephalides felis | 100 | | 100 | | 100 | | 99 | | 94 | | 74 |

**Tabelle 1b** Zeckenwirksamkeit des Mittels gemäß Beispiel 2 an der Katze

| | T0 | Appl. Vol | Parasit | W0 | | W1 | | W2 | | W3 | | W4 | | W5 |
|---|---|---|---|---|---|---|---|---|---|---|---|---|---|---|
| 1.Infestation Tag -4 | Behandlung | ml/kg | | T2 | 2.Infestation Tag 7 | T9 | 3.Infestation Tag 14 | T16 | 4.Infestation Tag 21 | T23 | 5.Infestation Tag 28 | T30 | 6.Infestation Tag 35 | T37 |
| | VB1 | 0.1 | Ixodes ricinus | 74 | | 99 | | 96 | | 72 | | 82 | | 89 |
| | VB2 | 0.15 | Ixodes ricinus | 84 | | 99 | | 92 | | 84 | | 73 | | 68 |
| | Beispiel 2 | 0.15 | Ixodes ricinus | 70 | | 100 | | 100 | | 97 | | 100 | | 95 |
| | VB3 | 0.1 | Ixodes ricinus | 71 | | 100 | | 100 | | 96 | | 93 | | 76 |

Appl. Vol. = Aufgetragenes Volumen in ml / kg Körpergewicht

„Wert“% = Wirksamkeit in %, berechnet über geometrische Mittelwertbestimmung gegenüber einer unbehandelten Kontrollgruppe

**Tabelle 2a** Flohwirksamkeit des Mittels gemäß Beispiel 2 am Hund

| 1.Infestation Tag -4 | 2. Infestation Tag -1 | T0 | Appl. Vol | Parasit | W0 | 3.Infestation Tag 7 | W1 | 4.Infestation Tag 14 | W2 | 5.Infestation Tag 21 | W3 | 6.Infestation Tag 28 | W4 | 7.Infestation Tag 35 | W5 | 8.Infestation Tag 42 | W6 | 9.Infestation Tag 49 | W7 |
|---|---|---|---|---|---|---|---|---|---|---|---|---|---|---|---|---|---|---|---|
| | | Behandlung | ml/kg | | T2 | | T9 | | T16 | | T23 | | T30 | | T37 | | T44 | | T51 |
| | | VB 1 | 0.1 | Ctenocephalides felis | 100 | | 100 | | 100 | | 99 | | 99 | | 100 | | 62 | | 33 |
| | | VB 2 | 0.15 | Ctenocephalides felis | 100 | | 100 | | 100 | | 100 | | 100 | | 99 | | 99 | | 76 |
| | | Beispiel 2 | 0.15 | Ctenocephalides felis | 100 | | 99 | | 100 | | 100 | | 100 | | 100 | | 100 | | 77 |
| | | VB 3 | 0.1 | Ctenocephalides felis | 100 | | 100 | | 100 | | 100 | | 98 | | 74 | | nd | | nd |

**Tabelle 2b** Zeckenwirksamkeit des Mittels gemäß Beispiel 2 am Hund

| 1.Infestation Tag -4 | 2. Infestation Tag -1 | T0 | Appl. Vol | Parasit | W0 | 3.Infestation Tag 7 | W1 | 4.Infestation Tag 14 | W2 | 5.Infestation Tag 21 | W3 | 6.Infestation Tag 28 | W4 | 7.Infestation Tag 35 | W5 | 8.Infestation Tag 42 | W6 | 9.Infestation Tag 49 | W7 |
|---|---|---|---|---|---|---|---|---|---|---|---|---|---|---|---|---|---|---|---|
| | | Behandlung | ml/kg | | T2 | | T9 | | T16 | | T23 | | T30 | | T37 | | T44 | | T51 |
| | | VB 1 | 0.1 | Rhipicephalus sanguineus | 97 | | 100 | | 100 | | 100 | | 99 | | 94 | | 93 | | 65 |
| | | VB 2 | 0.15 | Rhipicephalus sanguineus | 96 | | 100 | | 100 | | 100 | | 100 | | 99 | | 98 | | 74 |
| | | Beispiel 2 | 0.15 | Rhipicephalus sanguineus | 92 | | 100 | | 100 | | 100 | | 100 | | 99 | | 99 | | 88 |
| | | VB 3 | 0.1 | Rhipicephalus sanguineus | 60 | | 94 | | 99 | | 98 | | 98 | | 91 | | nd | | nd |

Appl. Vol. = Aufgetragenes Volumen in ml / kg Körpergewicht

„Wert"% = Wirksamkeit in %, berechnet über arithmetische Mittelwertbestimmung gegenüber einer unbehandelten Kontrollgruppe

**Tabelle 3** Zeckenwirksamkeit des Mittels gemäß Beispiel 2 am Hund

| 1.Infestation Tag -4 | T0 | Appl. Vol | Parasit | W0 | 2.Infestation Tag 7 | W1 | 3.Infestation Tag 14 | W2 | 4.Infestation Tag 21 | W3 | 5.Infestation Tag 28 | W4 |
|---|---|---|---|---|---|---|---|---|---|---|---|---|
| | Behandlung | ml/kg | | T2 | | T9 | | T16 | | T23 | | T30 |
| | VB1 | 0.15 | Dermacentor variabilis | 25 | | 98 | | 99 | | 100 | | 98 |
| | Beispiel 2 | 0.15 | Dermacentor variabilis | 55 | | 100 | | 99 | | 100 | | 100 |
| | VB3 | 0.1 | Dermacentor variabilis | 34 | | 89 | | 80 | | 66 | | 87 |

Appl. Vol. = Aufgetragenes Volumen in ml / kg Körpergewicht

„Wert"% = Wirksamkeit in %, berechnet über geometrische Mittelwertbestimmung gegenüber einer unbehandelten Kontrollgruppe

**Patentansprüche**

1. Mittel zur Bekämpfung von Parasiten an Tieren, enthaltend ein N-Arylpyrazol und ein Pyrethroid in einer Formulierung enthaltend:

   - ein aliphatisches, cyclisches Carbonat

- einen aliphatischen cyclischen oder acyclischen Polyether sowie
- einen oder mehrere Ester eines zwei- oder dreiwertigen Alkohols mit bis zu drei Kohlenstoffatomen mit organischen Fettsäuren mit 6 bis 18 Kohlenstoffatomen.

**2.** Mittel gemäß einem der vorstehenden Ansprüche, enthaltend 1 bis 27,5 Gew.-%. an Arylpyrazol.

**3.** Mittel gemäß einem der vorstehenden Ansprüche, enhaltend 10 bis 70 Gew.-% an aliphatischem, cyclischem Carbonat.

**4.** Mittel gemäß einem der vorstehenden Ansprüche, enhaltend 20 bis 77,5 Gew-% an aliphatischem cyclischem oder acyclischem Polyether.

**5.** Mittel gemäß einem der vorstehenden Ansprüche, enhaltend ein $\alpha$-Cyanopyrethroid

**6.** Mittel gemäß Anspruch 5, enthaltend alpha-Cypermethrin, Cyfluthrin, beta-Cyfluthrin, Cyhalothrin, Cypermethrin, Deltamethrin, Fenvalerate, Flucythrinate, Flumethrin oder tau-Fluvalinate.

**7.** Mittel gemäß einem der Ansprüche 1 bis 4, enthaltend ein Typ-1 Pyrethroid

**8.** Mittel gemäß Anspruch 7, enthaltend Allethrin, Bioallethrin, Permethrin, Phenothrin, Resmethrin, Tetramethrin oder Transfluthrin.

**9.** Mittel gemäß einem der Ansprüche 1 bis 4, enthaltend ein Nicht-Ester-Pyrethroid.

**10.** Mittel gemäß Anspruch 9, enthaltend Etofenprox, Halfenprox, Silafluofen.

**11.** Verwendung eines Mittels gemäß einem der vorstehenden Ansprüche zur Herstellung eines Arzneimittels zur Bekämpfung von Parasiten an Tieren.

## Claims

**1.** Composition for controlling parasites on animals, comprising an N-arylpyrazole and a pyrethroid in a formulation comprising:

- an aliphatic cyclic carbonate
- an aliphatic cyclic or acyclic polyether and
- one or more esters of a dihydric or trihydric alcohol having up to three carbon atoms with organic fatty acids having 6 to 18 carbon atoms.

**2.** Composition according to any of the preceding claims, comprising from 1 to 27.5% by weight of arylpyrazole.

**3.** Composition according to either of the preceding claims, comprising from 10 to 70% by weight of an aliphatic cyclic carbonate.

**4.** Composition according to any of the preceding claims, comprising from 20 to 77.5% by weight of an aliphatic cyclic or acyclic polyether.

**5.** Composition according to any of the preceding claims, comprising an $\alpha$-cyanopyrethroid.

**6.** Composition according to Claim 5, comprising alpha-cypermethrin, cyfluthrin, beta-cyfluthrin, cyhalothrin, cypermethrin, deltamethrin, fenvalerate, flucythrinate, flumethrin or tau-fluvalinate.

**7.** Composition according to any of Claims 1 to 4, comprising a type 1 pyrethroid.

**8.** Composition according to Claim 7, comprising allethrin, bioallethrin, permethrin, phenothrin, resmethrin, tetramethrin or transfluthrin.

9. Composition according to any of Claims 1 to 4, comprising a non-ester pyrethroid.

10. Composition according to Claim 9, comprising etofenprox, halfenprox, silafluofen.

11. Use of a composition according to any of the preceding claims for preparing a medicament for controlling parasites on animals.

## Revendications

1. Agent destiné à lutter contre les parasites chez les animaux, contenant un N-arylpyrazole et un pyréthroïde dans une formulation qui contient :

   - un carbonate aliphatique cyclique,
   - un polyéther aliphatique cyclique ou acyclique, et
   - un ou plusieurs esters d'un alcool bi- ou trivalent contenant jusqu'à trois atomes de carbone avec des acides gras organiques contenant 6 à 18 atomes de carbone.

2. Agent selon l'une quelconque des revendications précédentes, contenant 1 à 27,5 % en poids d'arylpyrazole.

3. Agent selon l'une quelconque des revendications précédentes, contenant 10 à 70 % en poids de carbonate aliphatique cyclique.

4. Agent selon l'une quelconque des revendications précédentes, contenant 20 à 77,5 % en poids de polyéther aliphatique cyclique ou acyclique.

5. Agent selon l'une quelconque des revendications précédentes, contenant un $\alpha$-cyanopyréthroïde.

6. Agent selon la revendication 5, contenant de l'alpha-cyperméthrine, de la cyfluthrine, de la bêta-cyfluthrine, de la cyhalothrine, de la cyperméthrine, de la deltaméthrine, du fenvalérate, du flucythrinate, de la fluméthrine ou du tau-fluvalinate.

7. Agent selon l'une quelconque des revendications 1 à 4, contenant un pyréthroïde de type 1.

8. Agent selon la revendication 7, contenant de l'alléthrine, de la bioalléthrine, de la perméthrine, de la phénothrine, de la resméthrine, de la tétraméthrine ou de la transfluthrine.

9. Agent selon l'une quelconque des revendications 1 à 4, contenant un pyréthroïde non-ester.

10. Agent selon la revendication 9, contenant de l'étofenprox, de l'halfenprox, du silafluofen.

11. Utilisation d'un agent selon l'une quelconque des revendications précédentes pour la fabrication d'un médicament destiné à lutter contre les parasites chez les animaux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 2006014802 A1
- WO 2005090313 A1
- FR 2834288 A1
- WO 09828277 A
- US 06069157 A
- WO 200031043 A
- DE 19824487
- WO 09804530 A
- WO 09962903 A
- EP 00933363 A
- EP 00911329 A
- WO 09856767 A
- US 05814652 A
- WO 09845274 A
- WO 9840359 A
- WO 09828279 A
- WO 09828278 A
- DE 19650197
- WO 09824767 A
- EP 00846686 A
- EP 00839809 A
- WO 09728126 A
- EP 00780378 A
- GB 02308365 A
- US 05629335 A
- WO 09639389 A
- US 05556873 A
- EP 00659745 A
- US 05321040 A
- EP 00511845 A
- EP 234119 A
- EP 295117 A
- WO 9824769 A
- WO 04098290 A
- WO 2001065941 A1
- EP 1013170 A1
- JP 11049618 A
- WO 9522902 A1
- FR 2713891 A1
- GB 2396557 A1
- WO 9616544 A2
- WO 2004098290 A1
- US 6395765 B
- WO 2005048713 A
- GB 2396557 A

### In der Beschreibung aufgeführte Nicht-Patentliteratur


- **HUNTER, J. S., III ; D. M. KEISTER ; P. JEANNIN.** Fipronil: A new compound for animal health. *Proc. Amer. Assoc. Vet. Parasitol. 39th Ann. Mtg.,* 1994, 48
- **BIRCKEL, P. ; BROMET-PETIT, M. ; BROMET, N. ; WEIL, A.** Skin distribution of fipronil by microautoradiography following topical administration to the beagle dog. Cochet, Pascal. *European Journal of Drug Metabolism and Pharmacokinetics,* 1997, vol. 22 (3), 211-216
- **MEIDAN, VICTOR M. ; BONNER, MICHAEL C. ; MICHNIAK, BOZENA B.** Transfollicular drug delivery - Is it a reality?. *International Journal of Pharmaceutics,* 2005, vol. 306 (1-2), 1-14
- **ENDRIS RG ; MATTHEWSON, COOKE D ; AMODIE D.** Repellency and efficacy of 65% permethrin and 9.7% fipronil against Ixodes ricinus. *Vet Therapeutics,* 2000, vol. 1 (3), 159-168
- **ENDRIS RG ; COOKE D ; AMODIE D ; SWEENWY DL ; KATZ TL.** Repellency and efficacy of 65% permethrin and selamectin spot-on formulations against Ixodes ricinus on dogs. *Vet Terapeutics.,* 2002, vol. 3 (1), 64-71
- **KOLACZINSKI, JAN ; CURTIS, CHRIS.** Laboratory evaluation of fipronil, a phenylpyrazole insecticide, against adult Anopheles (Diptera: Culicidae) and investigation of its possible cross-resistance with dieldrin in Anopheles stephensi. *London School of Hygiene and Tropical Medicine, London, UK. Pest Management Science,* 2001, vol. 57 (1), 41-45
- Antagonism of fipronil toxicity by piperonyl butoxide and S,S,S-tributyl phosphorotrithioate in the German cockroach (Dictyoptera: Blattellidae). **VALLES, STEVEN M. ; KOEHLER, PHILIP G. ; BRENNER, RICHARD J.** Journal of Economic Entomology. Center for Medical, Agricultural and Veterinary Entomology, 1997, vol. 90, 1254-1258